# EUROPEAN PATENT APPLICATION

(11) **EP 2 884 414 A1**
(43) Date of publication of application: **17.06.2015**
(21) Application number: 14197125.9
(22) Date of filing: 10.12.2014
(51) Int. Cl.: G06F 19/00, A61B 5/103, A61B 5/117, A61B 5/16, A61B 5/00

(54) **Health state determining method and apparatus using facial image**

(30) Priority: 11.12.2013 KR 20130153847
(71) Applicant: Korea Institute of Oriental Medicine, Daejeon 305-811 (KR)
(72) Inventor: Do, Jun Hyeong, 305-811 Daejeon (KR); Jang, Jun Su, 305-811 Daejeon (KR); Kim, Jong Yeol, 301-776 Daejeon (KR)
(74) Representative: advotec.

(57) **Abstract**

A health state determining method is disclosed, which includes receiving a facial image of a user, obtaining at least one user database (DB) image corresponding to the facial image, and determining a health state of the user by comparing the facial image to the at least one user DB image.

## Description

### CROSS-REFERENCE TO RELATED APPLICATION

This application claims the priority benefit of Korean Patent Application No. 10-2013-0153847, filed on December 11, 2013, in the Korean Intellectual Property Office, the disclosure of which is incorporated herein by reference.

### BACKGROUND

### 1. Field of the Invention

The present invention relates to a health state determining method and apparatus using a facial image.

### 2. Description of the Related Art

In modern society, a focus on health consciousness has been increasing. In line with the increasing focus on health consciousness, related technologies, for example, a data analysis method and tool based on real-time collection of data, have been developed and advanced. Thus, monitoring a state of health and receiving a personalized healthcare service are enabled.

In addition, customer demands have diversified and a level of expectation has increased due to a change in overall consumer consciousness. Thus, added emphasis is being placed on convenience and customization in using health services and related systems. For example, personalized healthcare businesses are performed based on data associated with health of individuals, for example, prevention of lifestyle related diseases and weight control programs, are experiencing rapid growth.

In the past, healthcare services were limited to treatment of diseases provided mainly to patients by hospitals or medical centers. However, the healthcare services presently encompass, for example, preventing diseases in advance and maintaining health, and are provided to healthy people.

Thus, consumer preferences for precautionary healthcare including scrutinizing or measuring a state of health and controlling an adequate amount of exercise are increasing in conjunction with improved standards of living and increasing interest in a quality of life and wellness.

### SUMMARY

According to embodiments of the present invention, there is provided a method and an apparatus that are conceived in response to recent industrial trends and may determine a state of health by comparing a captured facial image to a prestored user database (DB) image.

According to an aspect of the present invention, there is provided a health state determining method including receiving a facial image of a user, obtaining at least one user DB image corresponding to the facial image of the user, and determining a state of health of the user, hereinafter referred to as a health state of the user, by comparing the facial image of the user to the at least one user DB image.

The health state determining method may further include performing user authentication. The obtaining of the at least one user DB image may include obtaining at least one user DB image corresponding to authentication information associated with the user.

The performing of the user authentication may include applying a facial recognition algorithm to the facial image of the user and performing the user authentication based on a result of the applying.

The performing of the user authentication may include receiving the authentication information associated with the user, and performing the user authentication by comparing the authentication information associated with the user to predetermined authentication information.

The at least one user DB image may include at least one of a facial image corresponding to a healthy state of the user, a facial image corresponding to a semi-healthy state of the user, and a facial image corresponding to an ill state of the user.

The health state determining method may further include generating complexion information by performing color coordinate transformation on the facial image of the user and each of the at least one user DB image.

The determining of the health state of the user may include determining a similarity between the facial image of the user and each of the at least one user DB image based on a value of transformed color coordinates, and determining a health state corresponding to a user DB image having a highest similarity to be the health state of the user.

The determining of the health state of the user may include determining a general health state of the user based on a similarity between a global area of the facial image of the user and a global area of each of the at least one user DB image, or determining an organ based health state of the user based on a similarity between a local area of the facial image of the user and a local area of each of the at least one user DB image.

The determining of the health state of the user may include determining a difference between at least one local area of a facial image of the at least one user DB image corresponding to a healthy state and at least one local area of the facial image of the user, and determining that a health state of an organ corresponding to a local area in which the difference exceeds a predetermined threshold is deteriorated.

The health state determining method may further include storing the at least one user DB image.

The storing of the at least one user DB image may include receiving at least one facial image of the user, correcting the at least one facial image, receiving a health state corresponding to the at least one facial image, and storing, as the at least one user DB image, the corrected facial image and a health state corresponding to the corrected facial image.

The storing of the at least one user DB image may include receiving a plurality of chronological facial images of the user, applying a training model to the chronological facial images, and storing a user DB image based on a result of the applying of the training model.

The storing of the at least one user DB image may include storing at least one chronological user DB image, storing the at least one user DB image and a health state corresponding to the at least one user DB image, and outputting information on a chronological change in the health state of the user.

According to another aspect of the present invention, there is provided a health state determining apparatus including a capturer configured to capture a facial image of a user, a user DB configured to store at least one user DB image corresponding to the facial image of the user, and a health state determiner configured to determine a health state of the user by comparing the facial image of the user to the at least one user DB image.

The health state determining apparatus may further include an authenticator configured to perform user authentication. The health state determiner may obtain at least one user DB image corresponding to authentication information associated with the user and compare the obtained at least one user DB image to the facial image of the user.

The authenticator may apply a facial recognition algorithm to the facial image of the user and perform the user authentication based on a result of the applying.

The health state determining apparatus may further include an inputter to which the authentication information associated with the user is input. The authenticator may perform the user authentication by comparing the authentication information to predetermined authentication information.

The health state determining apparatus may further include a complexion information generator configured to generate complexion information by performing color coordinate transformation on the facial image of the user and each of the at least one user DB image.

The health state determiner may determine a similarity between the facial image of the user and each of the at least one user DB image based on a value of transformed color coordinates, and determine a health state corresponding to a user DB image having a highest similarity to be the health state of the user.

The health state determiner may determine a difference between at least one local area of a facial image of the at least one user DB image corresponding to a healthy state and at least one local area of the facial image of the user, and determine that a health state of an organ corresponding to a local area in which the difference exceeds a predetermined threshold value is deteriorated.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
FIG. 1 is a block diagram illustrating an example of a health state determining apparatus according to an embodiment of the present invention;
FIG. 2 is a diagram illustrating an example of an overall algorithm of a method of determining a health state of a user according to an embodiment of the present invention;
FIG. 3 is a block diagram illustrating an example of storing a user database (DB) image in a user DB according to an embodiment of the present invention;
FIGS. 4A through 4C are flowcharts illustrating examples of a health state determining method according to embodiments of the present invention;
FIG. 5 is a flowchart illustrating an example of storing a user DB image according to an embodiment of the present invention;
FIG. 6 illustrates an example of extracting a feature point from a color-corrected facial image according to an embodiment of the present invention;
FIG. 7 illustrates an example of a facial image including demarcated facial areas according to an embodiment of the present invention;
FIG. 8 is a flowchart illustrating an example of a user DB storing process according to an embodiment of the present invention;
FIG. 9 is a flowchart illustrating an example of a health state determining method according to an embodiment of the present invention; and
FIGS. 10A and 10B are graphs illustrating examples of a health restoration level according to an embodiment of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the accompanying drawings, however, the present invention is not limited thereto or restricted thereby.

When it is determined a detailed description related to a related known function or configuration that may make the purpose of the present invention unnecessarily ambiguous in describing the present invention, the detailed description will be omitted here. Also, terms used herein are defined to appropriately describe the exemplary embodiments of the present invention and thus may be changed depending on a user, the intent of an operator, or a custom. Accordingly, the terms must be defined based on the following overall description of this specification.

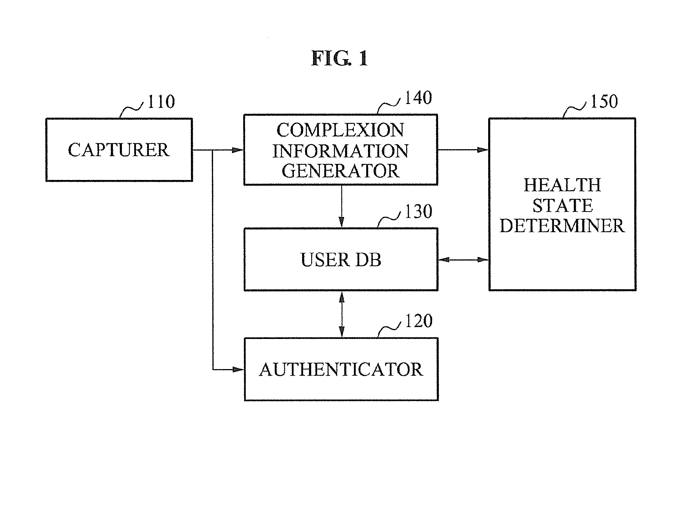

FIG. 1 is a block diagram illustrating an example of a health state determining apparatus according to an embodiment of the present invention.

Referring to FIG. 1, the health determining apparatus includes a capturer 110, an authenticator 120, a user database (DB) 130, a complexion information generator 140, and a health state determiner 150.

The capturer 110 capturers a facial image of a user. The capturer 110 may capture a static image or a video based on a control by a central processing unit (CPU). The capturer 110 may include an auxiliary light source, for example, a flash (not shown), to provide an amount of light required for the capturing. The capturer 110 may include various capturing devices such as a charge-coupled device (CCD) or a photodiode. The capturer 110 may be physically included in the health state determining apparatus. Alternatively, similarly to a digital camera or a camcorder, the capturer 110 may be physically independent from, yet electrically connected to, the health state determining apparatus.

Although not illustrated in FIG. 1, a corrector (not shown) corrects the captured image. For example, the corrector may remove an artifact and corrects a color modulated by reflected light. The corrector may correct a color of the input image.

The authenticator 120 performs user authentication.

In an example, the authenticator 120 may receive the facial image of the user from the capturer 110 and perform the user authentication based on the facial image of the user. For example, the authenticator 120 may apply a facial recognition program to the facial image of the user. The authenticator 120 may apply various facial recognition programs, for example, a principal component analysis (PCA), a linear discriminant analysis (LDA), elastic bunch graph matching, a hidden Markov model, multi-linear subspace learning, and neuronal motivated dynamic link matching. It may be easily understood by a person skilled in the related art that types of the facial recognition programs may not be limited thereto. The authenticator 120 identifies the user corresponding to the input facial image based on a result of facial recognition. The authenticator 120 may perform the user authentication using a verified user account. The performing of the user authentication based on a result of the applying of the facial recognition programs by the authenticator 120 may be provided as a simple illustration. Thus, it may be obvious that the authenticator 120 may perform user verification and authentication using a facial image based user identifying method, for example, an iris recognition method.

In another example, the authenticator 120 may receive authentication information from the user. For example, the health state determining apparatus may further include an inputter (not shown). The inputter may be provided in a form of a keypad or a touchscreen including a soft key, and receive the authentication information from the user and transmit the received authentication information to the authenticator 120. The authentication information may include at least one of a user identifier and a password. The authenticator 120 may perform the user authentication by comparing the input authentication information to prestored authentication information.

The user DB 130 stores a user DB image of each user. The user DB 130 includes a plurality of user DB images corresponding to different health states of each user. For example, the user DB 130 may include at least one user DB image as classified in Table 1.

**[Table 1]**

| | | |
|---|---|---|
| First user | Healthy | First image |
| | Semi-healthy | Second image |
| | Ill | Third image |
| Second user | Healthy | Fourth image |
| | Semi-healthy | Fifth image |
| | Ill | Sixth image |
| Third user | Healthy | Seventh image |
| | Semi-healthy | Eighth image |
| | Ill | Ninth image |

As indicated in Table 1, the user DB 130 may store a first image corresponding to a healthy state of a first user, a second image corresponding to a semi-healthy state of the first user, and a third image corresponding to an ill state of the first user. The user DB 130 may also store a fourth image corresponding to a healthy state of a second user, a fifth image corresponding to a semi-healthy state of the second user, and a sixth image corresponding to an ill state of the second user. Similarly, the user DB 130 may store a seventh image corresponding to a healthy state of a third user, an eighth image corresponding to a semi-healthy state of the third user, and a ninth image of an ill state of the third user. In Table 1, a classification standard, for example, the healthy state, the semi-healthy state, and the ill state, may be a simple illustrative example and thus, it may be obvious to a person skilled in the related art that various modifications may be applicable. In addition, although the user DB 130 is described in the foregoing to include the first through ninth images, the description may be provided as an illustrative example. The user DB 130 may store graphic data of the images described in the foregoing, or include only color information on each image. For example, the user DB 130 may include color coordinate data of the first through ninth images in lieu of the first through ninth images. The color coordinate data may be color coordinate information or statistically processed color coordinate information associated with each image. Alternatively, the color coordinate data may include color coordinate information on at least one local area of a face.

As indicated in Table 1, the storing of the images may be performed by classifying the images into the healthy state, the semi-healthy state, and the ill state. The classifying may be performed by receiving classification standards when storing a user DB image. For example, when an image is captured and stored in the user DB 130, information on which classification standard may be applied to the image may be further received. Thus, the user DB image may be stored based on the classification standards as indicated in Table 1.

Alternatively, when an image is input, the image may be stored by being automatically classified based on a predetermined standard. For example, the health state determining apparatus may apply a health classification function to the input image, and classify and store the input image based on a result of the applying of the health classification function. A detailed description will be provided hereinafter.

Also, the user DB image stored in the user DB 130 may be updated when a new image is input. Alternatively, a training unit (not shown) may store a more accurate user DB image by applying a training model to a user DB image to be input chronologically.

The training unit may store a user DB image improved by comparing input user DB images to one another. For example, the training unit may receive candidate user DB images, and compare the candidate user DB images to one another. The training unit may detect a user DB image improved based on a result of the comparing, and store the user DB image. The training unit may store, as the user DB image, a candidate user DB image that is most suitable for the training model among the candidate user DB images.

The complexion information generator 140 generates complexion information from the facial image input from the capturer 110. For example, the complexion information generator 140 may perform color coordinate transformation on the input facial image, and generate transformed color coordinate information for each pixel. Alternatively, the complexion information generator 140 may generate color coordinate information corresponding to each of at least one local area of a face. The complexion information generator 140 may generate the complexion information based on various color coordinates, for example, Yellow, Chrominance red, Chrominance blue (YCrCb), Red, Green, Blue (RGB), Hue, Saturation, Value (HSV), Hue, Saturation, Lightness (HSL), standard RGB (sRGB), Commission Internationale de l'Eclairage (CIE)-XYZ, CIE-Lab, and CIE-Luv, and types of the color coordinates may not be limited.

The health state determiner 150 compares the facial image captured by the capturer 110 to a prestored user DB image, and determines a health state based on a result of the comparing. The health state determiner 150 loads a user DB image corresponding to an authenticated user from the user DB 130. For example, when the user DB 130 includes the user DB images indicated in Table 1 and the second user performs the user authentication, the health state determiner 150 may load, from the user DB 130, the fourth through sixth images corresponding to the authenticated second user.

The health state determiner 150 may compare the captured facial image to the user DB image loaded from the user DB 130. For example, the health state determiner 150 may compare the captured facial image to each of the fourth through sixth images. The health state determiner 150 may directly compare the images, or compare the images based on the complexion information generated by the complexion information generator 140.

In an example, the health state determiner 150 may obtain a similarity by comparing the captured facial image to at least one corresponding user DB image. For example, the health state determiner 150 may obtain a similarity to each of the fourth through sixth images by comparing the captured facial image to each of the fourth through sixth images. The health state determiner 150 may obtain the similarity based on a correlation function between two comparison targets, but a method of measuring the similarity may not be limited thereto. The health state determiner 150 may obtain similarities as indicated in Table 2.

**[Table 2]**

| | Similarity to the fourth image | Similarity to the fifth image | Similarity to the sixth image |
|---|---|---|---|
| Similarity | 0.3 | 0.9 | 0.7 |

As indicated in Table 2, the health state determiner 150 may determine a health state corresponding to a highest similarity to be a current overall health state of a user. For example, in Table 2, based on the similarity to the fifth image being highest, the health state determiner 150 may determine the semi-healthy state to be the overall health state of the user and output a result of the determining. In an example, the health state determiner 150 may perform similarity determination on a global area of a face, and a result of performing the similarity determination may be indicated in Table 2. In another example, the health state determiner 150 may perform the similarity determination on each local area of the face, and a result of performing the similarity determination may be indicated in Table 3.

**[Table 3]**

| | Similarity to the fourth image | Similarity to the fifth image | Similarity to the sixth image |
|---|---|---|---|
| Similarity in a forehead area | 0.8 | 0.2 | 0.1 |
| Similarity in a cheek area | 0.2 | 0.9 | 0.8 |
| Similarity in a nose area | 0.1 | 0.9 | 0.7 |

The health state determiner 150 may obtain similarities indicated in Table 3, and determine a more detailed health state. The health state determiner 150 may determine a health state corresponding to a highest similarity in each area to be an organ based current health state of a user. Referring to Table 3, the similarity in the forehead area is highest in the fourth image, and the health state determiner 150 may determine that a first organ corresponding to the forehead area is healthy. Also, the similarity in the cheek area is highest in the fifth image, the health state determiner 150 may determine that a second organ corresponding to the cheek area is semi-healthy. Similarly, the similarity in the nose area is highest in the fifth image, the health state determiner 150 may determine that a third organ corresponding to the nose area is semi-healthy.

As described in the foregoing, in an example, the health state determiner 150 may determine the overall health state or the organ based health state of the user based on the similarity between the captured facial image and the user DB image.

In another example, the health state determiner 150 may compare an input facial image to a user DB image corresponding to a healthy state, and determine the health state of the user based on a result of the comparing. The health state determiner 150 may compare the input facial image to the user DB image corresponding to the healthy state, and determine a difference. For example, the health state determiner 150 may determine the difference based on a difference between color coordinate information on the input facial image and color coordinate information on the user DB image corresponding to the healthy state. When the determined difference exceeds a predetermined threshold, the health state determiner 150 may determine that an overall health state of the user is deteriorated. The health state determiner 150 may perform difference determination on a global area of a face, or on each local area of the face. Thus, the health state determiner 150 may determine the organ based health state corresponding to each local area of the face.

The health state determiner 150 may determine the organ based health state based on a constitution. For example, the health state determiner 150 may differently set a health state corresponding to the difference based on, for example, Taeyangin, Taeeumin, Soyangin, and Soeumin. Thus, despite detection of an identical difference, a health state of an organ of a Taeyangin physiological type person may be determined to be good, but a health state of the organ of a Taeeumin physiological type person may be determined to be deteriorating. The health state corresponding to the difference may be predetermined based on the constitution.

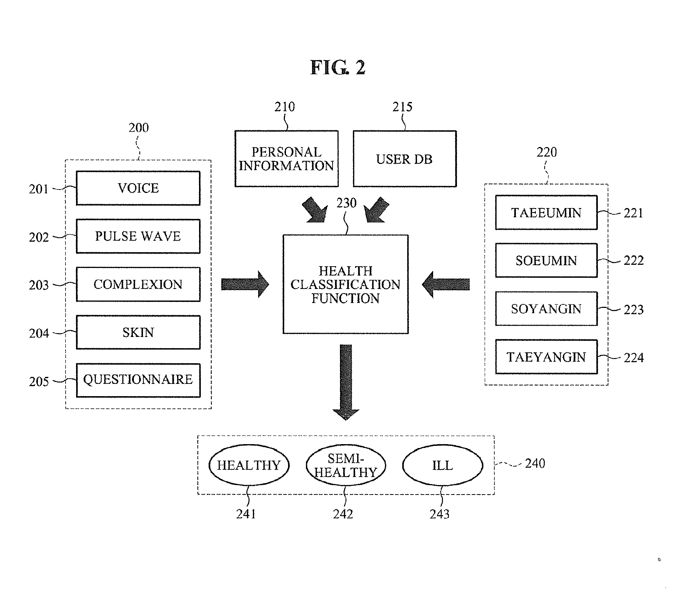

FIG. 2 is a diagram illustrating an example of an overall algorithm of a method of determining a health state of a user according to an embodiment of the present invention. FIG. 2 is provided to illustrate a health classification function 230 to be applied when classification of user DB images to be stored in a user DB 215 is automatically performed.

Referring to FIG. 2, the method of determining the health state of the user, also referred to as a health state determining method, includes receiving physical quantity information 200 on the user, personal information 210 on the user, and constitutional information 220 on the user to determine the health state of the user.

The personal information 210 may include personal details of the user including, for example, an age, a gender, a height, a weight, and a body mass index (BMI), an occupation, and an education level of the user.

The physical quantity information 200 may indicate values obtained by measuring physical elements of the user that are used as variables to determine healthiness of the user. For example, the physical quantity information 200 may include voice information 201 associated with a voice of the user, pulse wave information 202 associated with a pulse wave of the user, complexion information 203 associated with complexion, or a color of a face, of the user, skin information 204 associated with skin of the user, questionnaire information 205 obtained based on a health questionnaire completed by the user. Sets of the physical quantity information 200 may be input, to the health classification function 230, as a variable of the health classification function 230 to determine the healthiness of the user.

The health classification function 230 may be a function used to obtain information on the health state of the user by receiving the physical quantity information 200, the personal information 210, and the constitutional information 220, and determine the healthiness of the user.

The health classification function 230 may be generated using sets of clinical data. For example, the health state determining method may include calculating a functional equation by inputting plural sets of the clinical data to a classification model. The calculated functional equation may be used as the health classification function 230. The classification model may be at least one of a logistic regression analysis, a neural network analysis, a support vector machine analysis, a decision tree analysis, and a linear determinant analysis (LDA).

The health classification function 230 may be provided separately based on a constitution of a user. For example, the health classification function 230 may be provided as individual health classification functions for Taeeumin 221, Soeumin 222, Soyangin 223, and Taeyangin 224. The health state determining method may include selecting a health classification function based on a constitution of a user by receiving the constitutional information 220 on the user, and storing an image based on a classification standard in the user DB 215 using the selected health classification function.

The health state determining method may include classifying a health state 240 of the user into a healthy state 241, a semi-healthy state 242, and an ill state 243 based on input information. The health state determining method may include storing the user DB image in the user DB 215 based on the classifying.

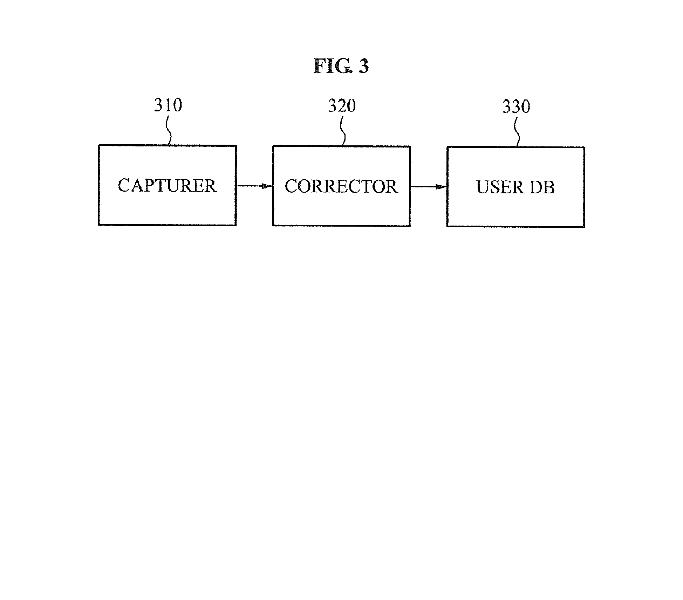

FIG. 3 is a block diagram illustrating an example of storing a user DB image in a user DB 330 according to an embodiment of the present invention.

Referring to FIG. 3, capturer 310 captures an image to generate a user DB image.

A corrector 320 corrects the captured image. For example, the corrector 320 may eliminate an artifact and correct a color modulated by reflected light.

The user DB 330 stores the corrected user DB image based on a classification standard.

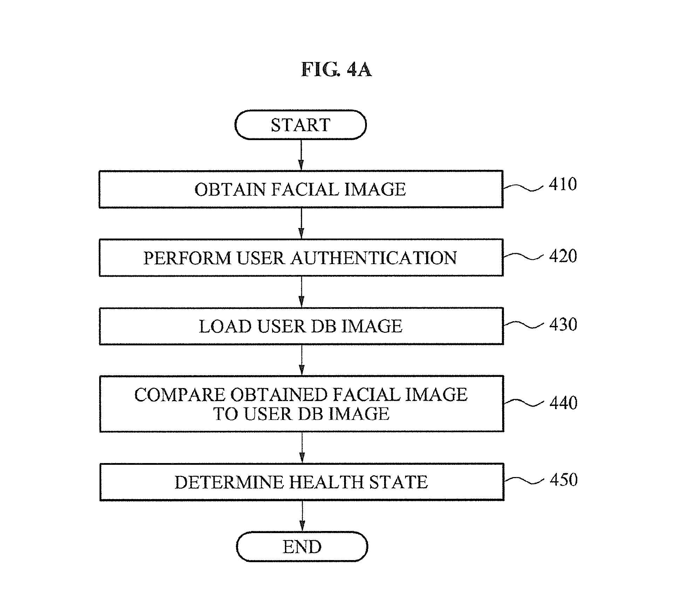

FIGS. 4A through 4C are flowcharts illustrating examples of a health state determining method according to embodiments of the present invention.

Referring to FIG. 4A, in operation 410, the health state determining method captures a facial image. For example, the health state determining method may capture the facial image directly using a capturer. Alternatively, the health state determining method may obtain the facial image. For example, the health state determining method may receive the facial image from a physically separated capturer. Alternatively, the health state determining method may obtain the facial image by loading a prestored facial image. Alternatively, the health state determining method may obtain the facial image by loading a facial image from a communicable external source.

In operation 420, the health state determining method performs user authentication. As described in the foregoing, the health state determining method may perform the user authentication based on the obtained facial image. Alternatively, the health state determining method may further receive authentication information and perform the user authentication based on the input authentication information.

In operation 430, the health state determining method loads a prestored user DB image corresponding to an authenticated user. The user DB image may include a facial image corresponding to a healthy state, a semi-healthy state, and an ill state of the user.

In operation 440, the health state determining method compares the obtained facial image to the user DB image. In operation 450, the health state determining method determines a health state of the user.

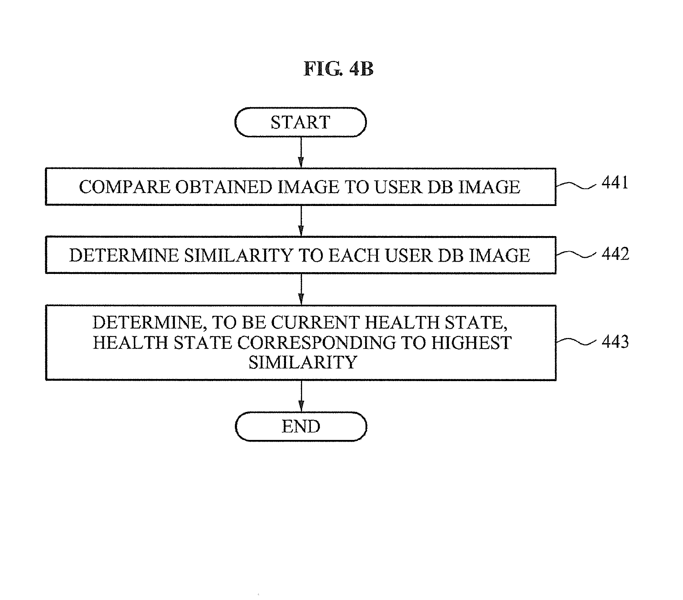

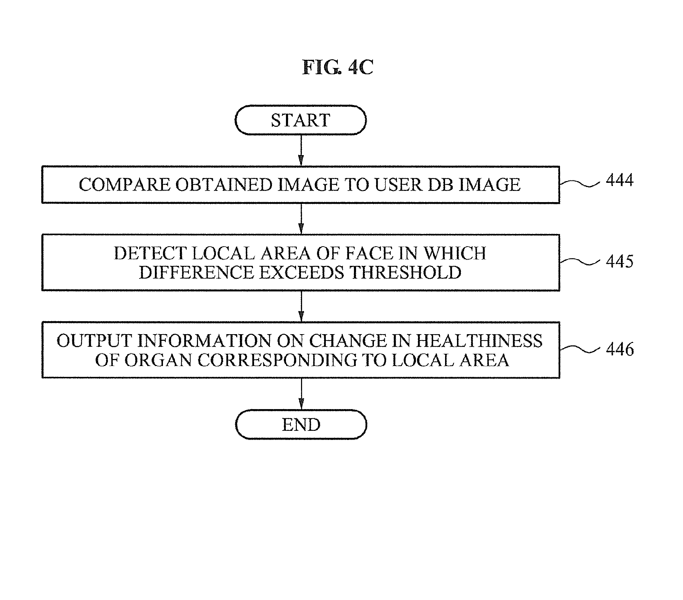

FIGS. 4B and 4C are flowcharts illustrating examples of comparing an obtained facial image to a user DB image.

Referring to FIG. 4B, in operation 441, a health state determining method compares an obtained image to a user DB image.

In operation 442, the health state determining method determines a similarity to each of the user DB image.

In operation 443, the health state determining method determines a health state having a highest similarity to be a current health state.

As described in the foregoing, the health state determining method may perform the comparing based on a global area of an image or a local area of the image.

Referring to FIG. 4C, in operation 444, a health state determining method compares an obtained facial image to a user DB image. The user DB image may correspond to a healthy state of a user.

In operation 445, the health state determining method detects a local area in which a difference exceeds a threshold among local areas of the facial image.

In operation 446, the health state determining method outputs information on a change in healthiness of an organ corresponding to a local area. For example, the health state determining method may determine that health of the organ corresponding to the local area in which the difference exceeds the threshold is deteriorated. In addition, the health state determining method may set a plurality of thresholds and detailed health states.

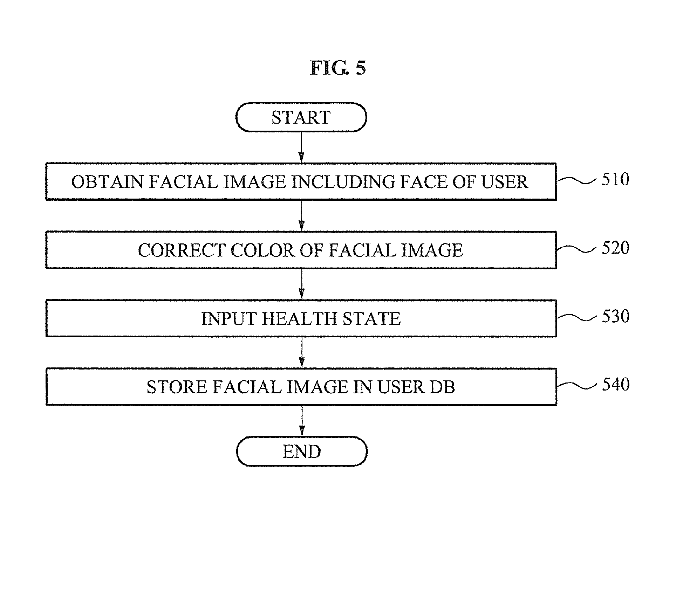

FIG. 5 is a flowchart illustrating an example of storing a user DB image according to an embodiment of the present invention.

Referring to FIG. 5, in operation 510, a health state determining method obtains a facial image including a face of a user.

In operation 520, the health state determining method corrects a color of the facial image. Alternatively, the health state determining method may further perform corrections, for example, elimination of an artifact, on the facial image.

In operation 530, the health state determining method receives a health state corresponding to the facial image. For example, the health state determining method may further receive information as to which classification standard the obtained facial image belongs, for example, a healthy state, a semi-healthy state, and an ill state.

In operation 540, the health state determining method stores, in a user DB, the facial image based on the input classification standard.

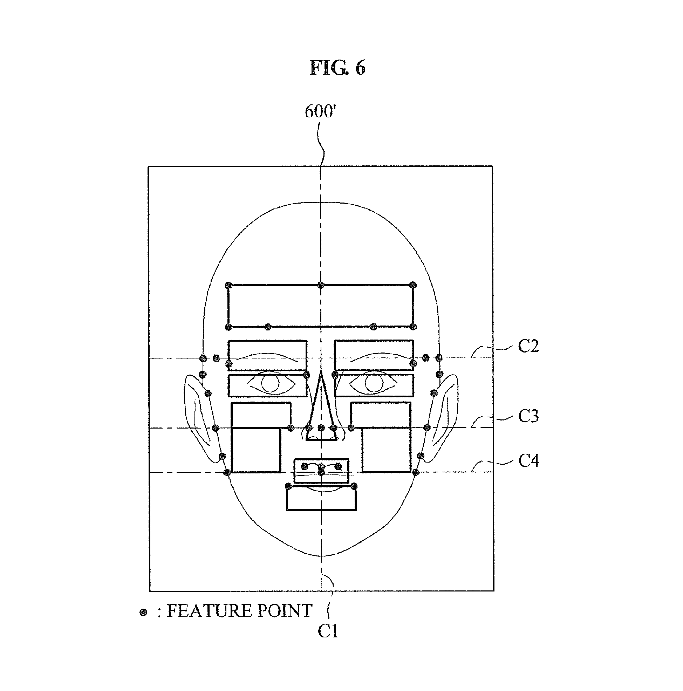

FIG. 6 illustrates an example of extracting a feature point (•) from a color-corrected facial image 600' according to an embodiment of the present invention. The feature point may be detected using a method of verifying a pixel value of each pixel included in the facial image 600' and extracting a boundary line and a contour line.

When the feature point is detected, a face center line (C1) connecting a center feature point of a forehead to a center feature point of lips, a first face demarcation line (C2) connecting feature points of edges of eyes, a second face demarcation line (C3) connecting a feature points of edges of earlobes to a feature point of a nasal tip, a third face demarcation line (C4) connecting feature points of edges of lips may be formed on the facial image 600'. The face center line C1 and the face demarcation lines C2 through C4 may be used to demarcate facial areas included in a face.

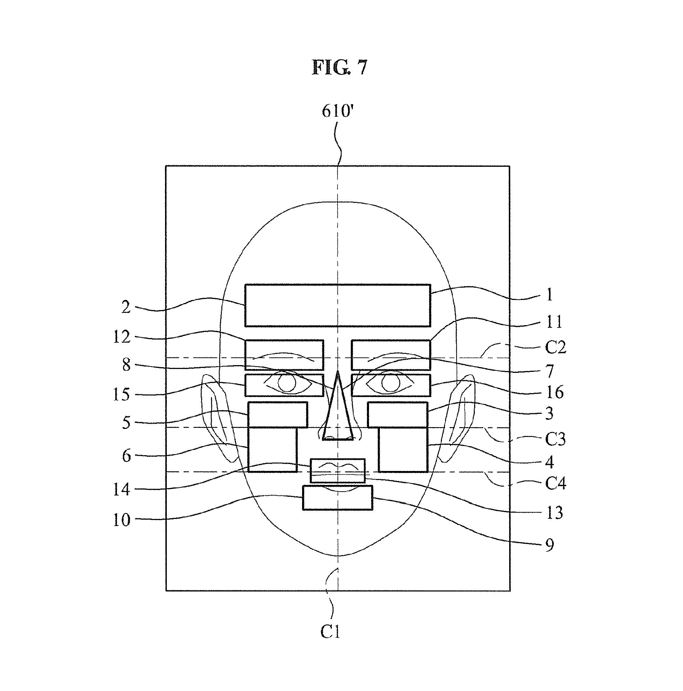

FIG. 7 illustrates an example of a facial image 610' including demarcated facial areas according to an embodiment of the present invention. Referring to FIG. 7, based on a face center line C1, a face may be divided into a left side and a right side. Also, based on first through third face demarcation lines C2 through C4, facial areas, for example, a forehead, an upper cheek, a lower cheek, a nose, and a jaw may be demarcated. Thus, the facial image 610' may be at least one of a left side forehead area (1), a right side forehead area (2), an entire forehead area (1 and 2), a left side upper cheek area (3), a left side lower cheek area (4), an entire left side cheek area (3 and 4), a right side upper cheek area (5), a right side lower cheek area (6), an entire right side cheek area (5 and 6), a left side nose area (7), a right side nose area (8), an entire nose area (7 and 8), a left side jaw area (9), a right side jaw area (10), an entire jaw area (9 and 10), a left side eye area (11), a right side eye area (12), a both eye area (11 and 12), a left side lower eye area (16), a right side lower eye area (16), a left side lip area (13), a right side lip area (14), and an entire lip area (13 and 14).

A complexion component of each facial area may be generated by detecting a color component of pixels from each facial area in the facial image 610', and calculating at least one of a mean of detected color components, a standard deviation, and a mode and a coefficient of variation (CV) in a histogram.

Thus, complexion information on an entire face may be generated by collecting and combining the generated complexion components.

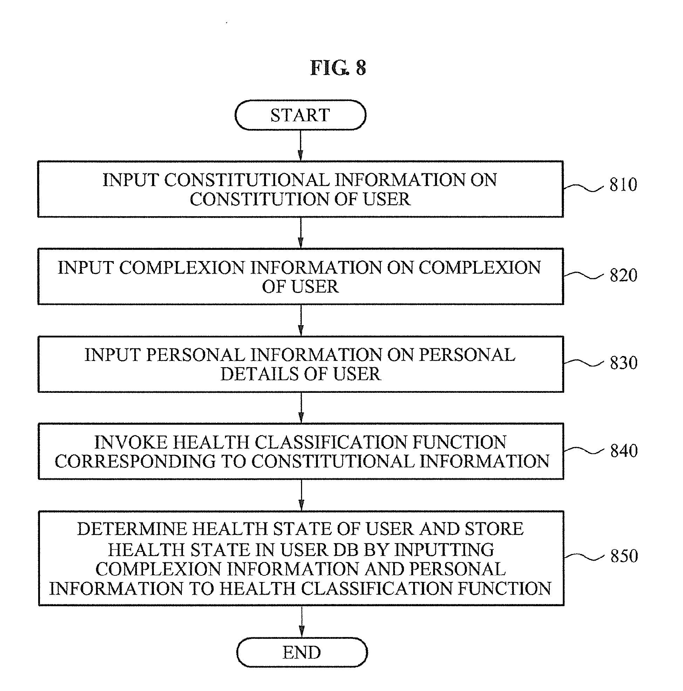

FIG. 8 is a flowchart illustrating an example of a user DB storing process according to an embodiment of the present invention.

Referring to FIG. 8, in operation 810, a health state determining method receives constitutional information on a constitution of a user. In operation 820, the health state determining method receives complexion information on a complexion of the user. In operation 830, the health state determining method receives personal information on personal details of the user.

In operation 840, the health state determining method invokes a health classification function corresponding to the constitutional information.

To perform operation 840, the health state determining method may generate the health classification function for each constitution. In detail, the health state determining method may receive plural sets of reference complexion information including a plurality of complexion components, and receive plural sets of clinical data corresponding to the plural sets of the reference complexion information. The complexion components may include a component indicating a characteristic of the complexion, for example, a brightness component, a red component, and a blue component. In addition, the clinical data may include at least one of constitutional information on a constitution of a clinical test subject, personal information on the clinical test subject, and health state information on a health state of the clinical test subject.

The plural sets of the reference complexion information and the plural sets of the clinical data may be classified based on the constitution to verify reference complexion information and clinical data shared by a Taeumin type person, a Soeumin type person, a Soyangin type person, and a Taeyangin type person.

Subsequently, a relationship between the complexion components and the health state may be calculated based on the constitution using the reference complexion information and the clinical data classified by the constitution and a classification model. The relationship between the complexion components and the health state may be an index indicating that a probability of a user having a certain constitution is healthy is high or low based on a complexion component of the user. The classification model used to calculate the relationship may be at least one of a logistic regression analysis, a neural network analysis, a support vector machine analysis, a decision tree analysis, and an LDA.

At least one major complexion component for each constitution may be extracted from the plurality of the complexion components using the reference complexion information and the clinical data classified by the constitution and the classification model. Through such an operation, the major complexion component used to readily indicate a health state based on the constitution may be extracted.

Subsequently, the health state determining method may generate the health classification function for each constitution using the extracted at least one major complexion component and the calculated relationship. Thus, the health state determining method may invoke the health classification function corresponding to the constitutional information input in operation 810 among the generated health classification function for each constitution.

In operation 850, the health state determining method determines a health state of the user by inputting the complexion information and the personal information to the invoked health classification function, and stores the determined health state in the user DB based on the classification standard.

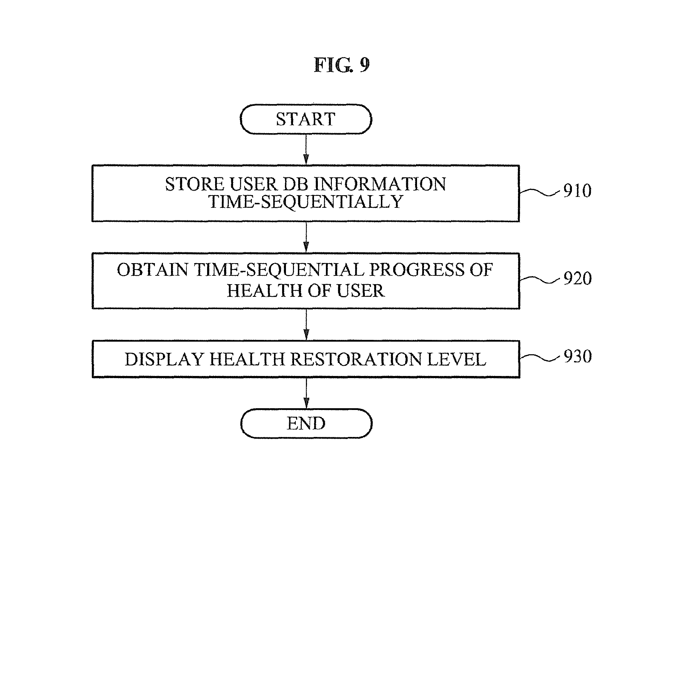

FIG. 9 is a flowchart illustrating an example of a health state determining method according to an embodiment of the present invention.

Referring to FIG. 9, in operation 910, the health state determining method chronologically stores user DB information. For example, the health state determining method may store an overall chronological health state of a user or an organ based chronological health state of the user.

In operation 920, the health state determining method obtains a chronological progress in health of the user.

In operation 930, the health state determining method chronologically displays a health restoration level. Thus, the user may easily understand a chronological change in health restoration or deterioration. In addition, the health state determining method may obtain the health restoration level based on the chronological change in the health state and further display the obtained health restoration level.

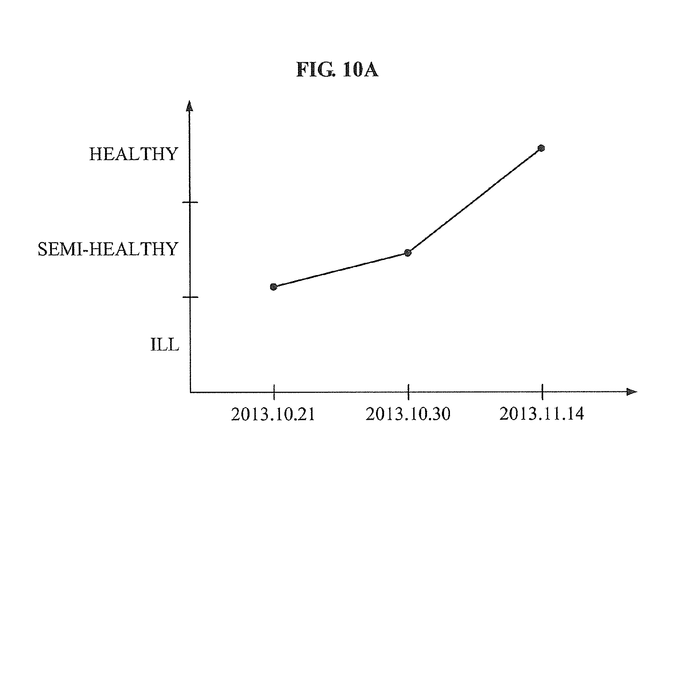

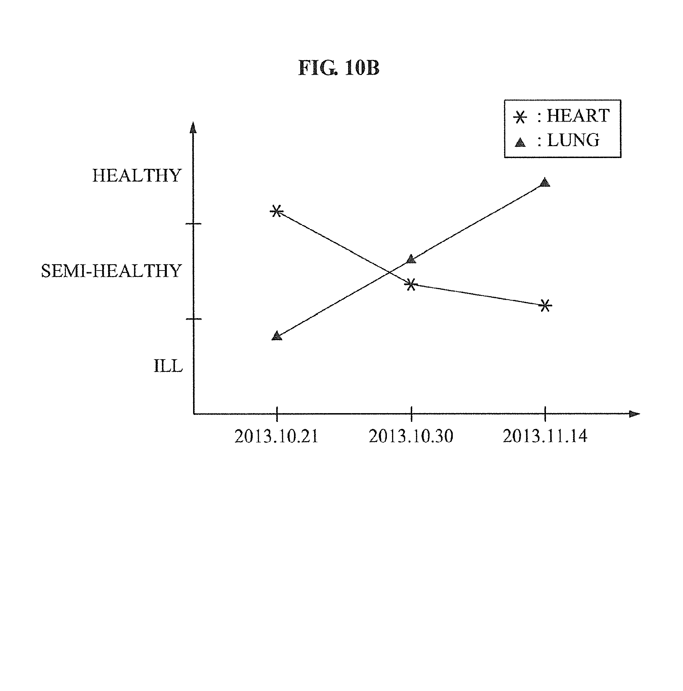

FIGS. 10A and 10B are graphs illustrating examples of a health restoration level according to an embodiment of the present invention.

FIG. 10A is a graph illustrating an example of a health restoration level according to an embodiment of the present invention.

A health state determining method may output a graph associated with chronological user DB information. The graph of FIG. 10A indicates chronological information on an overall health state of a user. The user may verify a progress of overall health of the user based on the graph.

FIG. 10B is a graph illustrating another example of a health restoration level according to an embodiment of the present invention.

A health state determining method may output a graph associated with chronological user DB information. The graph of FIG. 10B indicates chronological information on an organ based health state of a user. For example, information on a health state of a heart and a health state of a lung may be indicated as different graphs as illustrated in FIG. 10B. Thus, the user may verify a progress of health of each organ of the user based on the graphs.

According to example embodiments, there is provided a health state determining method and apparatus that may determine a health state of a user by comparing a captured facial image of the user to a prestored user DB image.

The units described herein may be implemented using hardware components and software components. For example, the hardware components may include microphones, amplifiers, band-pass filters, audio to digital convertors, and processing devices. A processing device may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

The software may include a computer program, a piece of code, an instruction, or some combination thereof, to independently or collectively instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums. The non-transitory computer readable recording medium may include any data storage device that can store data which can be thereafter read by a computer system or processing device. Examples of the non-transitory computer readable recording medium include read-only memory (ROM), random-access memory (RAM), CD-ROMs, magnetic tapes, floppy disks, optical data storage devices. Also, functional programs, codes, and code segments that accomplish the examples disclosed herein can be easily construed by programmers skilled in the art to which the examples pertain based on and using the flow diagrams and block diagrams of the figures and their corresponding descriptions as provided herein.

While this disclosure includes specific examples, it will be apparent to one of ordinary skill in the art that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents. Therefore, the scope of the disclosure is defined not by the detailed description, but by the claims and their equivalents, and all variations within the scope of the claims and their equivalents are to be construed as being included in the disclosure.

## Claims

1. A health state determining method, comprising:
receiving a facial image of a user;
obtaining at least one user database (DB) image corresponding to the facial image of the user; and
determining a health state of the user by comparing the facial image of the user to the at least one user DB image.

2. The method of claim 1, further comprising:
performing user authentication, and
wherein the performing of the user authentication comprises:
applying a face recognition algorithm to the facial image of the user or comparing the authentication information associated with the user to predetermined authentication information; and
performing the user authentication based on a result of the applying or of the comparing,
wherein the obtaining of the at least one user DB image comprises obtaining at least one user DB image corresponding to authentication information associated with the user.

3. The method of claim 1, wherein the at least one user DB image comprises at least one of a facial image corresponding to a healthy state of the user, a facial image corresponding to a semi-healthy state of the user, and a facial image corresponding to an ill state of the user.

4. The method of claim 1, further comprising:
correcting a color of the facial image of the user.

5. The method of claim 1, further comprising:
generating complexion information by performing color coordinate transformation on the facial image of the user and each of the at least one user DB image,
wherein the determining of the health state of the user comprises:
determining a similarity between the facial image of the user and each of the at least one user DB image based on a value of transformed color coordinates; and
determining a health state corresponding to a user DB image having a highest similarity to be the health state of the user.

6. The method of claim 5, wherein the determining of the health state of the user comprises:
determining a general health state of the user based on a similarity between a global area of the facial image of the user and a global area of each of the at least one user DB image, or determining an organ based health state of the user based on a similarity between a local area of the facial image of the user and a local area of each of the at least one user DB image.

7. The method of claim 1, further comprising:
generating complexion information by performing color coordinate transformation on the facial image of the user and each of the at least one user DB image,
wherein the determining of the health state of the user comprises:
determining a difference between at least one local area of a facial image of the at least one user DB image corresponding to a healthy state and at least one local area of the facial image of the user; and
determining that a health state of an organ corresponding to a local area in which the difference exceeds a predetermined threshold is deteriorated.

8. The method of claim 7, wherein the determining of the health state of the organ to be deteriorated comprises:
determining whether the health state is deteriorated by comparing the difference to a threshold predetermined based on a constitution.

9. The method of claim 1, further comprising:
storing the at least one user DB image,
wherein the storing of the at least one user DB image comprises:
receiving at least one facial image of the user;
correcting the at least one facial image;
receiving a health state corresponding to the at least one facial image; and
storing, as the at least one user DB image, the corrected facial image and a health state corresponding to the corrected facial image.

10. The method of claim 1, further comprising:
storing the at least one user DB image,
wherein the storing of the at least one user DB image comprises:
receiving a plurality of facial images of the user;
applying a training model to the facial images; and
storing a user DB image based on a result of the applying of the training model.

11. The method of claim 1, further comprising:
storing the at least one user DB image,
wherein the storing of the at least one user DB image comprises:
storing at least one chronological user DB image;
storing the at least one user DB image and a health state corresponding to the at least one user DB image; and
displaying a chronological change in the health state; and
displaying a health restoration level based on the chronological change in the health state.

12. The method of claim 1, further comprising:
storing the at least one user DB image,
wherein the storing of the at least one user DB image comprises:
receiving at least one facial image of the user;
applying a health classification function to the at least one facial image; and
storing, as the at least one user DB image, a health state corresponding to a result of the applying of the health classification function.

13. The method of claim 12, wherein the applying of the health classification function comprises:
applying, to the at least one facial image of the user, a health classification function corresponding to a constitution of the user.

14. A health state determining apparatus, comprising:
a capturer configured to capture a facial image of a user;
a user database (DB) configured to store at least one user DB image corresponding to the facial image of the user; and
a health state determiner configured to determine a health state of the user by comparing the facial image of the user to the at least one user DB image.

15. The apparatus of claim 14, further comprising:
a complexion information generator configured to generate complexion information by performing color coordinate transformation on the facial image of the user and each of the at least one user DB image.
